# EUROPÄISCHE PATENTSCHRIFT

(11) **EP 1 273 277 B1**
(45) Veröffentlichungstag und Bekanntmachung des Hinweises auf die Patenterteilung: **11.05.2005**
(21) Anmeldenummer: 02014705.4
(22) Anmeldetag: 03.07.2002
(51) Int. Cl.: A61F 7/10, A41D 13/12

(54) **Bekleidungsstück und Anordnung zum Kühlen eines unter Multipler Sklerose leidenden Patienten**
Clothing article with cooling means for a patient suffering from multiple sclerosis
Vêtement avec dispositif de refroidissement pour patient souffrant de sclérose en plaques

(30) Priorität: 03.07.2001 DE 10132102
(43) Veröffentlichungstag der Anmeldung: 08.01.2003
(73) Patentinhaber: med-s GmbH, 48151 Münster (DE)
(72) Erfinder: Moj, Jens, 48341 Altenberge (DE)
(74) Vertreter: Habbel, Lutz, Dipl.-Ing.

(56) Entgegenhaltungen:
- WO-A-01/17470
- US-A- 3 744 053
- US-A- 4 718 429
- US-A- 4 998 415
- US-A- 5 269 369
- US-A- 5 980 561

## Beschreibung

Die Erfindung betrifft ein Bekleidungsstück sowie eine Anordnung zum Kühlen eines Patienten, welcher unter Multipler Sklerose leidet, sowie die Verwendung eines kühlbaren Bekleidungsstückes zur Therapie eines derartigen Patienten.

Aus der gattungsbildenden US-A-4 998 415, US-A-4 718 429, DE 299 12 196 U1 sowie DE 200 02 182 U1 sind jeweils Kühlwesten bekannt, die als Teil der Arbeitskleidung Wärmestaus in Schutzanzügen vermeiden sollen. Die US-A-3 744 053 zeigt einen kompletten Klimatisierungsanzug mit Ober- und Unterteil, Handschuhen und Kapuze, der beispielsweise für Taucher in kalten Gewässern mit einem wärmenden und für Feuerwehrleute mit einem kühlenden Fluid durchströmt werden kann. Einen Hinweis auf eine medizinische Verwendbarkeit enthalten diese Druckschriften nicht.

Aus der DE 87 15 804 U1 sind handschuh-, socken-, manschettenartige und ähnlich ausgestaltete Vorrichtungen aus beschickbaren Hohlkörpern zum Kühlen von Körperpartien bekannt, und. Einen Hinweis auf eine Verwendbarkeit bei Multipler Sklerose enthält sie nicht, insbesondere nicht auf eine westen- oder jackenartige Ausgestaltung.

Das Wärmegefühl scheint bei Multipler Sklerose mit dem Auftreten eines Krankheitsschubes, also mit einem Fortschritt der Krankheit und einer dementsprechenden dauerhaften Verschlechterung der Befindlichkeit des Patienten einherzugehen. Durch die Begrenzung oder Unterbindung des Wärmeschubes wird nicht nur die momentane Befindlichkeit des Patienten verbessert sondern versucht, die dauerhafte Befindlichkeit des Patienten beizubehalten und den Krankheitsfortschritt einzudämmen oder zu unterbinden.

Der Erfindung liegt die Aufgabe zugrunde, anzugeben, wie die auftretenden Wärmeschübe von an Multipler Sklerose leidenden Patienten möglichst wirksam eingegrenzt oder sogar unterbunden werden können sowie eine dazu geeignete Anordnung unter Verwendung eines geeigneten Hilfsmittels anzugeben.

Diese Aufgabe wird durch ein Bekleidungsstück mit den Merkmalen des Anspruches 1 sowie durch eine Anordnung mit den Merkmalen des Anspruches 8 oder des Anspruches 9 oder durch die Verwendung eines Bekleidungsstückes gemäß Anspruch 13 oder 14 gelöst.

Die Erfindung schlägt mit anderen Worten zur Therapie des Patienten ein Bekleidungsstück mit darin angeordneten, als Schläuche ausgestalteten Kühlelementen vor, welche ihre Kühlwirkung an der Innenseite des Bekleidungsstückes entfalten. Es können also beispielsweise an der Außenseite des Bekleidungsstückes oder zwischen Außenseite und einem Futter des Bekleidungsstückes Schläuche vorgesehen sein, die an der Innenseite des Bekleidungselementes den Patienten kühlen. Gegebenenfalls kann eine zur Außenseite gerichtete Isolierung vorgesehen sein, die Kälteverluste einzuschränken hilft.

Es hat sich herausgestellt, dass insbesondere eine westenartige Ausgestaltung des Bekleidungsstückes ausreicht, um eine wirksame Eingrenzung des Wärmeschubes zu ermöglichen. Die Kühlung der Extremitäten, also der Beine und der Arme, ist von nachrangiger Bedeutung. Das Kühlmittel weist innerhalb des Bekleidungsstückes dort seine niedrigste Temperatur auf, wo es vorgekühlt in das Bekleidungsstück hineingeführt wird, also im Bereich der Hinfluß-Leitungen. Diese verlaufen vorschlagsgemäß im Rückenteil des Bekleidungsstückes, nahe der Wirbelsäule, beispielsweise bis in den Nackenbereich und bis zum Kopf des Patienten. Durch die Körpertemperatur des Patienten wird das Kühlmittel dabei erwärmt, so dass nach dieser primären Kühlwirkung der Rückfluß des Kühlmittels zu Stellen des Bekleidungsstückes geleitet werden kann, wo eine geringere Kühlwirkung erforderlich ist.

Da die Kühlelemente als Schläuche ausgestaltet sind, die mit dem Kältemedium gefüllt sind bzw. von dem Kältemedium durchströmt werden, ermöglichen sie die optimale Anpassung an die unterschiedlichsten Konturierungen von Bekleidungsstücken ohne die Verwendung speziell angefertigter Zuschnitte, sondern vielmehr unter Verwendung des preisgünstigen Vormaterials in Form von Schlauchmaterial.

Es kann vorteilhaft vorgesehen sein, einen Kühlkreislauf aufzubauen, um über längere Zeit eine gleichmäßige Kühlwirkung zu erzielen, die - im Vergleich zu vorgekühlten "Kühlakkus" - ein unangenehmes, zu starkes anfängliches Kältegefühl sicher ausschließen kann und die andererseits auch über einen längeren Zeitraum eine ausreichende Kühlwirkung sicherstellen kann.

Vorteilhaft kann das Bekleidungsstück optimal eng anliegend ausgestaltet sein. Um teure Maßanfertigungen zu vermeiden, kann hierzu eine Verstellbarkeit des Bekleidungsstückes vorgesehen sein. Unterschiedliche Wärmeeinwirkungen sind dabei jedoch möglichst zu vermeiden, so dass beispielsweise keine Faltenbildung in dem gekühlten Bereich des Bekleidungsstückes erfolgen sollte. Daher kann vorteilhaft vorgesehen sein, Vorder- und Rückenteil zu kühlen und dazwischen entweder eine ungekühlte Zone als Dehnungsausgleich vorzusehen, oder Vorderund Rückenteil an der Seite gar nicht miteinander zu verbinden. Im seitlichen Bereich des Bekleidungsstückes können dann verstellbare Verbindungselemente in Form z. B. handelsüblicher Verschlußelemente vorgesehen sein, beispielsweise horizontal verlaufende Knopfleisten, Häkchenverschlüsse od. dgl., so dass das Bekleidungsstück dem Patienten eng anliegend eingestellt werden kann. Wenn dabei zwischen Vorder- und Rückenteil ein seitlicher ungekühlter Bereich verbleibt bzw. entsteht, so ist dies, wie Untersuchungen gezeigt haben, unkritisch.

Abgesehen von der vorteilhaften Verstellmöglichkeit kann auch aus Gründen einer möglichst preisgünstigen Fertigung vorgesehen sein, Vorder- und Rückenteil des Bekleidungsstückes seitlich nicht miteinander zu verbinden. So kann ein Zuschnitt erzielt werden, bei dem Rücken, Schulter und Vorderteil einstückig ausgestaltet sind und besonders problemlos mit den Kühlelementen, beispielsweise den vorerwähnten Schläuchen, versehen werden können, indem diese durchgehend an den Vorder- und Rückenteilen verlaufen.

Durch die Möglichkeit, das Bekleidungsstück zwischen Vorder- und Rückenteil auftrennen zu können, ergibt sich zudem die vorteilhafte Möglichkeit, das Bekleidungsstück als flache Kühldecke zu verwenden. So kann der Patient es auf eine Weise tragen, z. B. sich auf- oder umlegen, die ihm das angenehmste Kälteempfinden oder die wirkungsvollste Linderung von Beschwerden ermöglicht, ohne dass eine bestimmte Tragweise bzw. Anordnung des Bekleidungsstückes vorgegeben ist, wie dies bei der Ausbildung als Weste der Fall ist.

Die Kühlwirkung ist insbesondere im Bereich von körperoberflächennahen Nervenbahnen vorteilhaft. Gegebenenfalls kann daher lediglich eine bereichsweise Anordnung der Kühlelemente am Bekleidungsstück vorgesehen sein.

Weiterhin kann aus diesem Grund auch vorgesehen sein, am Bekleidungsstück einen hoch aufragenden Kragen vorzusehen, der den Nacken- und evtl. auch Halsbereich des Patienten kühlt. Insbesondere kann vorgesehen sein, eine den Kopf des Patienten kühlende Kapuze vorzusehen, wobei diese entweder unmittelbar am Bekleidungsstück vorgesehen sein kann oder als abtrennbarer oder stets abgetrennter Bestandteil des Bekleidungsstückes ausgestaltet ist. In diesem zweiten Fall ist ein besonders einfacher Anschluß der Kapuze an die Kühlversorgung dadurch möglich, dass am westen- oder jackenartigen Bekleidungsstück Anschlüsse vorgesehen sind, die den Anschluß der Kapuze und damit die Einbindung der Kapuze in einen Kühlkreislauf ermöglichen.

Vorteilhaft kann der Rückfluß des Kühlmittels im vorderen Rumpfbereich des Patienten erfolgen, auch wenn aus rein therapeutischer Sicht die Kühlung im Rückenbereich des Patienten vordringlich ist. Durch eine Vergleichmäßigung der Kühlwirkung wird das Wohlbefinden des Patienten verbessert, da keine lokale und begrenzte Kühlwirkung erfolgt, die als unangenehm empfunden wird. Daher kann der Rückfluß vorteilhaft über den vorderen Rumpfbereich des Patienten erfolgen, wo die verringerte Kühlleistung immerhin zu einer Vergleichmäßigung des Temperaturempfindens beitragen kann im Vergleich zu einer Situation, bei der ausschließlich der Rücken des Patienten gekühlt wird.

Eine besonders preisgünstige Anordnung zum Kühlen des Patienten unter Verwendung des Bekleidungsstückes wird dadurch ermöglicht, dass eine gekühlte Flüssigkeit aus einem Isolierbehälter umgepumpt wird und durch die Kühlelemente des Bekleidungsstückes strömt. Hierzu kann eine kleine, preisgünstige Pumpe vorgesehen sein, deren Förderleistung regelbar ist.

Die regelbare Förderleistung ermöglicht dem Patienten die individuell angenehme Einstellung der Kühlleistung, so dass eine vergleichsweise tief heruntergekühlte Flüssigkeit im Isolierbehälter bevorratet werden kann, welche eine ausreichend lange Kühlversorgung sicherstellt.

Um eine unangenehm starke Kühlwirkung zu vermeiden, kann vorgesehen sein, dem rückfließenden, erwärmten Kühlfluid etwas von dem im Kühlbehälter befindlichen Fluid zuzumischen und diese so temperierte Fluidmischung dem Bekleidungsstück zuzuleiten.

Da nur ein vergleichsweiser geringer Energiebedarf für die Pumpe besteht, kann eine derartige Anordnung problemlos mit einer Betriebsspannung von 12 V betrieben werden, so dass sie dem Patienten ein vergleichsweise hohes Maß an Mobilität ermöglicht, z. B. in Fahrzeugen, Booten oder dergleichen.

Eine Alternative für eine derartige Anordnung kann dadurch vorgesehen sein, dass ein eigenes Kälteerzeugungsaggregat vorgesehen ist, wie dies beispielsweise aus dem Bereich von Kühlschränken bekannt ist. Über einen an sich bekannten Wärmetauscher kann das das Bekleidungsstück durchströmende Kühlmedium mittels dieses Kälteerzeugungsaggregates heruntergekühlt werden. Im einfachsten Fall kann das Kälteerzeugungsaggregat an eine vorgesehene Kühlleistung angepasst sein. Da das Kälteerzeugungsaggregat über lange Zeit betrieben werden kann, beispielsweise über mehrere Stunden, welche ein Wärmeschub dauert, kann diese Kühlleistung so gering bemessen sein, dass die Temperatur im Wärmetauscher des Kälteerzeugungsaggregates höher ist als beispielsweise in dem vorgenannten, alternativ zu verwendenden Isolierbehälter. Es kann daher vorgesehen sein, bei der Verwendung eines Kälteerzeugungsaggregates keine weiteren Regel- oder Eingriffsmöglichkeiten zu schaffen, so dass die Handhabung für den Patienten äußerst einfach darin besteht, das Kälteerzeugungsaggregat mit elektrischer Energie zu versorgen und ggf. separat auch noch einzuschalten.

Je nach Auslegung des Kälteerzeugungsaggregates kann auch eine derartige Anordnung mit 12 V betrieben werden und die vorgenannte Mobilität des Patienten ermöglichen.

Es kann jedoch auch bei Verwendung eines Kälteerzeugungsaggregates vorgesehen sein, die Kühlwirkung für den Patienten einstellbar zu machen. Hierzu kann ggf. die Leistung des Kälteerzeugungsaggregates geregelt werden oder die Förderleistung einer Pumpe, welche das Kühlmedium durch den Kühlkreislauf fördert.

Die Krankheit MS bewirkt beim Patienten eine teilweise Lähmung bzw. Disfunktion bestimmter Körperareale, wie Beine, Arme, Gehirnregionen, Augen, Rücken. Diese Lähmungen treten im Zusammenhang mit akuten Beschwerden auf, die entweder temporär oder langanhaltend sein können. Die Beschwerden, die gemäß des Krankheitsverlaufs in Schüben auftreten, führen neben erheblichen Schmerzen auch zur Immobilität des Patienten.

Dem Krankheitsbild liegt ein autoimmunologischer (Gen)- Defekt zugrunde, der die körpereigenen Leukozyten dazu veranlasst, eigene Zellen, speziell Nervenzellen zu zerstören. Im Rahmen dieser autoimmunologischen Abwehrreaktion spielt die körpereigene Produktion von NO2 (Stickstoff) eine Rolle.

Ziel der Therapie mit dem vorgeschlagenen, kühlbaren Bekleidungsstück ist es, durch gezielte Kälteeinwirkung an den entsprechenden Beschwerde-Lokalisationen eine Verminderung der körpereigenen Stickstoffproduktion zu erreichen, die wiederum eine deutlich reduzierte Schädigung der betroffenen Nervenzellgewebe nach sich zieht.

Zur Reduktion der Beschwerden ist es im akuten Stadium erforderlich, also unmittelbar bei Eintreten der Schübe, für einen bestimmten Zeitraum wie z.B. ca. 60 bis 120 Min. pro Tag eine gezielte und gesteuerte Kühlung zu applizieren. Dies bedeutet, dass speziell im Rücken- als auch im Kopfbereich des Patienten eine Kühlung erfolgen sollte, die sich auch auf den vorderen Rumpfbereich erstrecken kann.

Mit dem vorgeschlagenen kühlbaren Bekleidungsstück steht ein Applikationsmittel zur Verfügung, mit welchem eine ganz gezielte und auch steuerbare Kühltherapie möglich ist. Sowohl durch die verwendeten-Materialien als auch insbesondere durch deren Anordnung ist ein homogener Kühleffekt bei den bevorzugten Körperarealen erreichbar.

Bei der Anordnung des Bekleidungsstückes als Kühldecke, durch Auftrennen von Vorder- und Rückenteil, können vorteilhaft Polytraumata - Patienten therapiert werden. Bei diesen besteht aufgrund der erlittenen schweren Verletzungen ein hochgradiges akutes Schockrisiko. Dieser Schockzustand ist lebensbedrohlich und stellt in der Notfallmedizin eine große Komplikation dar.

Ziel des als "Kühldecke" ausgebreiteten kühlbaren Bekleidungsstückes ist es, zur Verringerung des Schockzustandes und mithin der Vermeidung des schockbedingten Todes des Patienten zu sorgen.

Dies wird dadurch erreicht, dass die direkte Umfeldtemperatur des Patienten gezielt und steuerbar reduziert wird. Dies erfolgt vorteilhaft durch mittels in die Decke eingearbeitete Schläuche, durch die gekühltes Wasser als unkompliziertes und nicht toxisches Kühlmediumgeführt wird,

Ein Ausführungsbeispiel der Erfindung wird anhand der Zeichnungen nachfolgend näher erläutert. Dabei zeigt
- Fig. 1: eine Ansicht von vorne auf eine Kühlweste und
- Fig. 2: eine Ansicht auf die Kühlweste von Fig. 1 mit einem hochgeschlagenen Vorderteil.

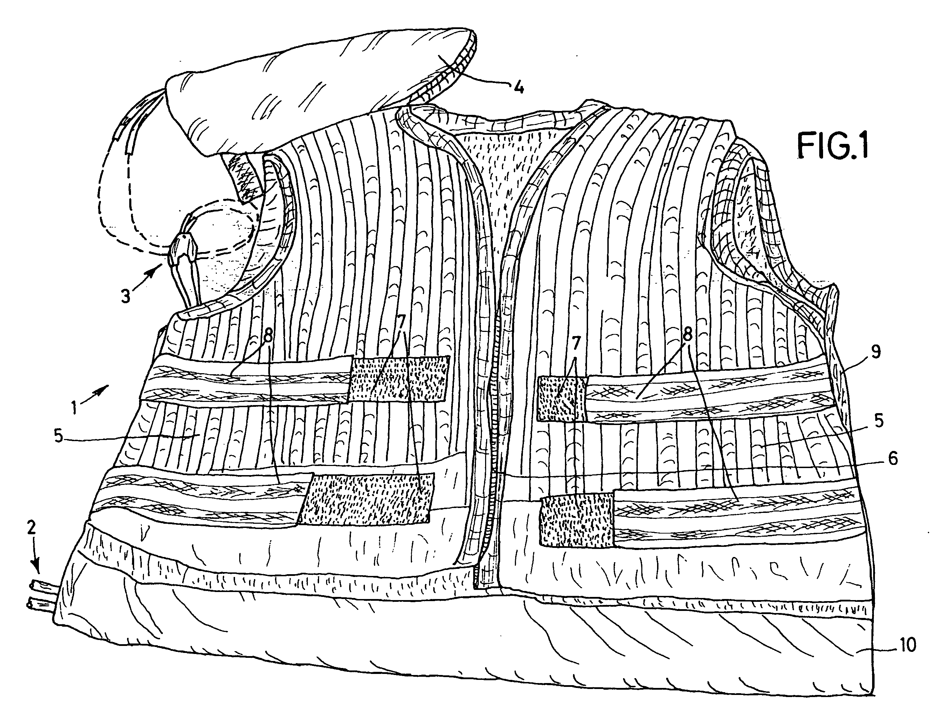

In Fig. 1 ist mit 1 ein Bekleidungsstück bezeichnet, welches als Kühlweste ausgestaltet ist. Das Bekleidungsstück 1 weist Anschlüsse 2 an ein Kühlaggregat auf, so dass ein Kühlkreislauf aufgebaut werden kann und ein gekühltes flüssiges Medium vom Kühlaggregat in das Bekleidungsstück 1 geleitet wird, während erwärmtes Kühlmedium durch die Anschlüsse 2 zum Kühlaggregat geleitet und dort abgekühlt wird.

Das Bekleidungsstück 1 weist weiterhin Anschlüsse 3 auf zum Anschluß an eine Kapuze 4, so dass auch die Kapuze 4 in den Kühlkreislauf eingebunden ist.

Das Bekleidungsstück 1 weist zwei Vorderteile 5 auf, die durch einen Reißverschluß 6 miteinander verbindbar sind und die auf ihrer Vorderseite jeweils zwei Halteflächen für einen Häkchenverschluß aufweisen, wobei die zugehörigen Halteriegel an einem Rückenteil 9 des Bekleidungsstückes 1 befestigt sind. Die Halteflächen 7 und Halteriegel 8 ermöglichen eine stufenlose und individuelle Anpassung des Bekleidungsstückes 1 an den Körperumfang des einzelnen Patienten, und die Anordnung zweier derartiger Verbindungselemente übereinander ermöglicht eine besonders gute Anpassung des Bekleidungsstückes 1 an den Patienten, so dass eine optimale Anlage des Bekleidungsstückes 1 und damit eine optimale Einwirkung der Kühlwirkung auf den Patienten ermöglicht wird.

Das Rückenteil 9 ist tiefer gezogen als die beiden Vorderteile 5 und weist einen unteren Saum 10 auf, in welchen die Anschlüsse 2 münden. Innerhalb des Saumes 10 ist eine Verteilung mit mehreren Abzweigungen vorgesehen, so dass das Kühlmedium von den Anschlüssen 2 auf verschiedene Kühlleitungsstränge innerhalb des Bekleidungsstückes 1 verzweigt wird. Zur Reduzierung der erforderlichen Pumpenleistung kann die Anzahl der Abzweigungen möglichst gering gehalten werden. Zugunsten einer möglichst gleichmäßigen Verteilung der Kühlwirkung und insbesondere dann, wenn strömungswiderstandsarme Abzweigungen verwendbar sind, kann die Anzahl der Abzweigungen möglichst hoch gewählt werden.

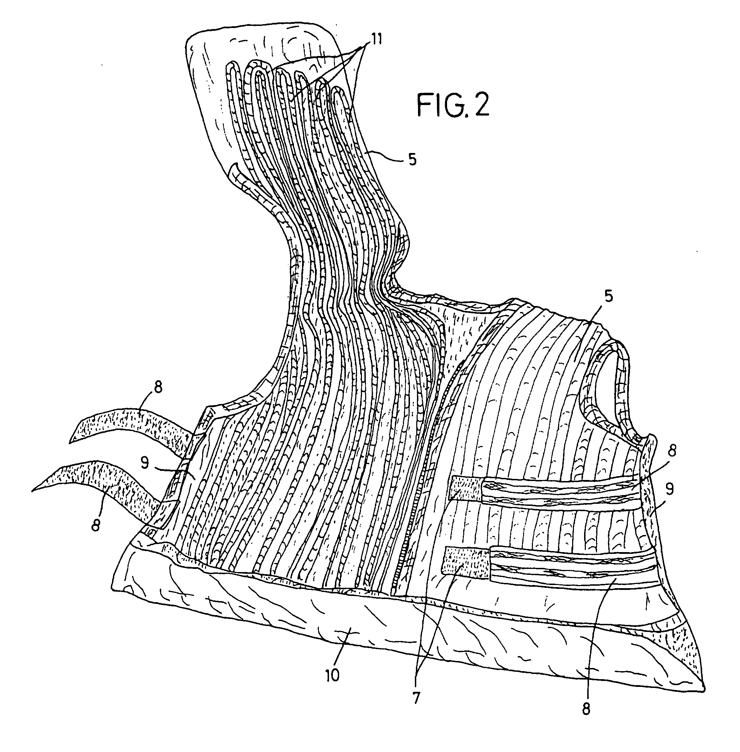

Fig. 2 zeigt, dass die Kühlelemente als Schläuche 11 ausgebildet sind, die durchgängig vom Rückenteil 9 über die Vorderteile 5 und zurück zum Saum 10 des Rückenteiles 9 verlaufen.

Die beiden Vorderteile 5 stoßen stets in der selben Weise aneinander an, wenn der Patient die Kühlweste angelegt hat; bei dem dargestellten Ausführungsbeispiel mittels eines Reißverschlusses 6. Zur Anpassung an den Körperumfang des Patienten dienen die Halteflächen 7 und die Halteriegel 8, so dass Überlappungen des Bekleidungsstückes 1 und damit Zonen unterschiedlich starker Kühlwirkung vermieden werden. Diese ist zwar grundsätzlich zulässig, kann aber das Wohlbefinden des Patienten beeinträchtigen, so dass aus diesem Grund Stellen mit zu starker Kühlwirkung unerwünscht sind.

Im seitlichen Bereich sind die Vorder- und Rückenteile 5 und 9 der Kühlweste nicht miteinander verbunden, sondern lediglich über die Halteflächen 7 und Halteriegel 8 miteinander verbindbar. Es könnte abweichend von dem dargestellten Ausführungsbeispiel eine Verbindung in Form eines Textilstreifens vorgesehen sein, der in Falten gelegt werden kann und den entsprechenden Ausgleich für den Umfang des Bekleidungsstückes 1 ermöglicht und der in diesen unterschiedlichen Zuständen festgelegt werden kann, beispielsweise auch mittels Häkchenverschlüssen.

Abweichend von dem dargestellten Ausführungsbeispiel kann vorgesehen sein, eine Kapuze fest an dem Bekleidungsstück 1 vorzusehen und auf diese Weise zuverlässig eine Kühlung des Nacken-, Hals- und Kopfbereiches des Patienten sicherzustellen. Alternativ kann vorgesehen sein, abweichend von dem dargestellten Ausführungsbeispiel keine Kühlkapuze und dementsprechend auch keine Anschlüsse für eine derartige Kapuze vorzusehen. Als weitere Alternative kann eine Kapuze mit eigenen Anschlüssen für eine Verbindung unmittelbar mit dem Kühlaggregat vorgesehen sein, so dass die Kühlwirkung der Kapuze ggf. getrennt von der Kühlwirkung des restlichen Bekleidungsstückes beeinflusst wereden kann.

Wenn abweichend von dem dargestellten Ausführungsbeispiel kein regelrechter Kühlkreislauf vorgesehen ist, also die Verwendung von als "Kühlakkus" bezeichneten Kühlelementen vorgesehen sind, die in sich geschlossen sind und nicht an ein Kälteaggregat angeschlossen werden, so kann insbesondere vorgesehen sein, Kühlelemente zu verwenden, die mit einem Latentwärmespeicher arbeiten, beispielsweise mit Wachsteilchen. In diesem Fall kann eine Kühlwirkung über vergleichsweise lange Zeit ermöglicht werden, ohne zu Anfang eine für den Patienten unangenehm niedrige Temperatur einstellen zu müssen.

## Patentansprüche

1. Westen- oder jackenartiges Bekleidungsstück (1) zur Therapie von an Multipler Sklerose leidenden Patienten, mit als Schläuche (11) ausgestalteten Kühlelementen, welche an der Innenseite des Bekleidungsstückes (1) eine Kühlwirkung entfalten,
und mit Anschlüssen (2) die einen Hin- und Rückfluß des Kühlmediums von bzw. zu einem externen Kühlaggregat ermöglichen, welches die Kühlelemente mit einem Kühlmedium speist,
**dadurch gekennzeichnet, dass**
die den Kühlmittel-Hinfluß führenden Schläuche (11) im wirbelsäulennahen Bereich des Rückenteils (9) des Bekleidungsstückes (1) angeordnet sind.

2. Bekleidungsstück nach Anspruch 1, **dadurch gekennzeichnet,**
**dass** Vorder- und Rückenteil (5, 9) des Bekleidungsstückes (1) über seitliche Verbindungselemente an unterschiedliche Körperumfänge der Patienten anpassbar sind,
wobei Vorder- und Rückenteil (5, 9) jeweils unverändert bleiben.

3. Bekleidungsstück nach Anspruch 1 oder 2, **dadurch gekennzeichnet, dass** die Schläuche (11) durchgehend entlang den Vorder- und Rückenteilen (5, 9) verlaufen.

4. Bekleidungsstück nach einem der vorhergehenden Ansprüche, **gekennzeichnet durch** eine Kapuze (4), welche in den Kühlkreislauf eingebunden ist.

5. Bekleidungsstück nach Anspruch 4, **dadurch gekennzeichnet, dass** die Kapuze (4) von dem Bekleidungsstück (1) trennbar oder getrennt ist und eine Unterbrechung des Kühlkreislaufs ermöglichende Anschlüsse (3) aufweist, die mit korrespondierenden Anschlüssen (3) des restlichen Bekleidungsstückes (1) zusammenwirken.

6. Bekleidungsstück nach einem der vorhergehenden Ansprüche, **dadurch gekennzeichnet, dass** die den Kühlmittel-Rückfluß führenden Schläuche (11) im Vorderteil (5) des Bekleidungsstückes (1) angeordnet sind.

7. Bekleidungsstück nach einem der vorhergehenden Ansprüche, **dadurch gekennzeichnet, dass** das Bekleidungsstück (1) zwischen Vorder- und Rückenteil (5, 9) auftrennbar ist, derart, dass das Bekleidungsstück (1) eine flache Kühldecke bildet.

8. Anordnung zum Kühlen eines an Multipler Sklerose leidenden Patienten,
mit einem Bekleidungsstück (1) nach einem der vorhergehenden Ansprüche,
wobei das Kühlaggregat durch einen Isolierbehälter gebildet ist, welcher eine gekühlte Flüssigkeit enthält,
und wobei der Isolierbehälter Anschlüsse zur Verbindung mit den Anschlüssen (2) des Bekleidungsstückes (1) aufweist,
und wobei in den Kühlkreislauf eine Pumpe mit regelbarer Förderleistung eingebunden ist.

9. Anordnung zum Kühlen eines an Multipler Sklerose leidenden Patienten,
mit einem Bekleidungsstück (1) nach einem der Ansprüche 1 bis 7,
wobei das Kühlaggregat ein elektrisch betriebenes Kälteerzeugungsaggregat aufweist, welches Anschlüsse zur Verbindung mit den Anschlüssen (2) des Bekleidungsstückes (1) aufweist.

10. Anordnung nach Anspruch 9, **dadurch gekennzeichnet, dass** die Leistung des Kälteerzeugungsaggregates regelbar ist.

11. Anordnung nach Anspruch 9 oder 10, **dadurch gekennzeichnet, dass** in den Kühlkreislauf eine Pumpe mit regelbarer Förderleistung eingeschaltet ist.

12. Anordnung nach einem der Ansprüche 9 bis 11, **dadurch gekennzeichnet, dass** elektrische Verbraucher wie eine Pumpe oder ein Kälteerzeugungsaggregat mit einer Betriebsspannung von 12 Volt betreibbar sind.

## Claims

1. Waistcoat-like or jacket-like garment (1) for the treatment of patients suffering from multiple sclerosis, having cooling elements designed as hoses (11), which provide a cooling effect on the inside of the garment (1), and with connections (2) permitting the inward and outward flow of the coolant from or to an external cooling unit, which feeds the cooling elements with a coolant, **characterised in that** the hoses (11) carrying the inward coolant flow are arranged in the region of the back portion (9) of the garment (1) close to the spine.

2. Garment according to claim 1, **characterised in that** the front and rear portions (5, 9) of the garment (1) may be adjusted to different physical sizes of patient by means of lateral binding elements, whereby the front and rear portions (5, 9) each remain unaltered.

3. Garment according to claim 1 or 2, **characterised in that** the hoses (11) run continuously along the front and rear portions (5, 9).

4. Garment according to one of the previous claims, **characterised in that** it has a hood (4) which is linked to the cooling circuit.

5. Garment according to claim 4, **characterised in that** the hood (4) is separable or separate from the garment (1) and has connections (3) permitting interruption of the cooling circuit, which interact with corresponding connections (3) of the remaining garment (1).

6. Garment according to one of the previous claims, **characterised in that** the hoses (11) carrying the coolant outward flow are arranged in the front portion (5) of the garment (1).

7. Garment according to one of the previous claims, **characterised in that** the garment (1) is separable between the front and rear portions (5, 9), such that the garment (1) forms a flat cooling blanket.

8. Arrangement for cooling a patient suffering from multiple sclerosis, having a garment (1) according to one of the previous claims, whereby the cooling unit comprises an insulated container containing a cooled liquid and whereby the insulated container has connections for linking to the connections (2) of the garment (1), and whereby a pump with controllable pumping performance is linked into the cooling circuit.

9. Arrangement for cooling a patient suffering from multiple sclerosis, having a garment (1) according to one of the claims 1 to 7, whereby the cooling unit has an electrically powered refrigerating unit which has connections for linking to the connections (2) of the garment (1).

10. Arrangement according to claim 9, **characterised in that** the output of the refrigerating unit is controllable.

11. Arrangement according to claim 9 or 10, **characterised in that** a pump of controllable output is connected into the cooling circuit.

12. Arrangement according to one of the claims 9 to 11, **characterised in that** electrical consumers such as a pump or a refrigerating unit may be operated at an operating voltage of 12 Volt.

## Revendications

1. Vêtement (1) en forme de gilet ou de veste pour le traitement de patients souffrant de sclérose en plaques, avec des éléments de refroidissement conçus comme des tuyaux (11) qui produisent un effet de refroidissement à l'intérieur du vêtement (1) et avec des raccords (2) permettant une arrivée et une évacuation de fluide réfrigérant respectivement depuis et vers un groupe de refroidissement externe qui alimente les éléments de refroidissement en fluide de refroidissement, **caractérisé en ce que** les tuyaux (11) acheminant l'arrivée de fluide de refroidissement sont disposés dans la partie proche de la colonne vertébrale du dos (9) du vêtement (1).

2. Vêtement selon la revendication 1, **caractérisé en ce que** le devant et le dos (5, 9) du vêtement (1) sont adaptables par des éléments de liaison latéraux à différentes corpulences de patients, le devant et le dos (5, 9) restant inchangés.

3. Vêtement selon la revendication 1 ou 2, **caractérisé en ce que** les tuyaux (11) sont continus le long des parties de devant et de dos (5, 9).

4. Vêtement selon l'une des revendications précédentes, **caractérisé en ce qu'**il comporte une capuche (4) intégrée dans le circuit de refroidissement.

5. Vêtement selon la revendication 4, **caractérisé en ce que** la capuche (4) peut être séparée ou est séparée du vêtement (1) et présente des raccords (3) permettant d'interrompre le circuit de refroidissement et coopérant avec des raccords (3) correspondants sur le reste du vêtement (1).

6. Vêtement selon l'une des revendications précédentes, **caractérisé en ce que** les tuyaux (11) acheminant le retour de fluide de refroidissement sont disposées dans le devant (5) du vêtement (1).

7. Vêtement selon l'une des revendications précédentes, **caractérisé en ce que** le vêtement (1) peut être séparé entre le devant et le dos (5, 9) de sorte que le vêtement (1) forme une couverture réfrigérante plate.

8. Dispositif destiné à rafraîchir un patient souffrant de sclérose en plaques, avec un vêtement (1) selon l'une des revendications précédentes, dans lequel le groupe de refroidissement est formé par un récipient isolant qui contient un liquide refroidi, dans lequel le récipient isolant possède des raccords destinés à être assemblés avec les raccords (2) du vêtement (1), et dans lequel une pompe à débit réglable est intégrée dans le circuit de refroidissement.

9. Dispositif destiné à rafraîchir un patient souffrant de sclérose en plaques, avec un vetement selon l'une des revendications 1 à 7, dans lequel le groupe de refroidissement est un groupe réfrigérant électrique qui possède des raccords destinés à être assemblés avec les raccords (2) du vêtement (1).

10. Dispositif selon la revendication 9, **caractérisé en ce que** la puissance du groupe réfrigérant est réglable.

11. Dispositif selon la revendication 9 ou 10, **caractérisé en ce qu'**une pompe à débit réglable est intégrée dans le circuit de refroidissement.

12. Dispositif selon l'une des revendications 9 à 11, **caractérisé en ce que** les consommateurs électriques, tels qu'une pompe ou un groupe réfrigérant, sont alimentés sous une tension de 12 volts.
